# EUROPEAN PATENT SPECIFICATION

(11) **EP 1 225 945 B1**
(45) Date of publication and mention of the grant of the patent: **13.07.2005**
(21) Application number: 00972477.4
(22) Date of filing: 03.11.2000
(51) Int. Cl.: A61M 16/06, A61M 16/00

(54) **MASK**
MASKE
MASQUE

(30) Priority: 03.11.1999 AU PQ382299
(43) Date of publication of application: 31.07.2002
(73) Proprietor: Australian Centre for Advanced Medicine Technology Ltd., Sydney, NSW 2006 (AU)
(72) Inventor: SULLIVAN, Colin, University of Sydney, NSW 2006 (AU); WILKIE, Paul, Australian Centre for Adv. Medical, Dept. of Medicine, University of Sydney, (AU)
(74) Representative: Faulkner, Thomas John
(86) International application number: PCT/AU2000/001349
(87) International publication number: WO 2001/032250

(56) References cited:
- WO-A-96/17643
- WO-A1-98/18514
- AU-A- 4 247 699
- US-A- 5 243 971
- US-A- 5 419 317
- US-A- 5 560 354
- US-A- 5 657 752
- US-A- 5 746 201
- US-A- 5 921 239

## Description

### Field of the Invention

This invention relates to a mask for supplying gases, typically air or oxygen to the airways of humans. The mask is particularly, but not exclusively, suited to infants, neonates, and premature neonates.

### Background of the Invention

Various masks are used to provide fresh air or oxygen to the airways of humans. A specialised category of masks is used to provide positive pressure to the human airway. Positive pressure applied in this manner has two different goals.

In a first category, positive pressure is applied to the lungs for the purpose of stabilising the lungs, and in particular for maintaining a minimum inflation level of the small air spaces in which gas transfer occurs (the alveoli). This therapy is very useful in patients with a variety of lung diseases, where the disease processes tend to lead to collapse (closure of the airway containing regions of the lung).

In a second category, the positive pressure is applied to the nasal airway with the intention of maintaining the pressure in, and the patency of, the upper airway. This form of positive airway pressure is known as nasal continuous positive airway pressure (nasal CPAP). This is now the "gold standard" treatment for the condition known as obstructive sleep apnea (OSA), and also for snoring and a variant of this therapy, bi-level positive pressure, is used to both stabilise the upper airway and provide additional positive pressure to support breathing. Obstructive sleep apnea is a condition in which the upper airway closes in sleep, and does so repeatedly Nasal CPAP, when applied for the duration of sleep, stabilises the upper airway and allows for normal sleep and normal breathing.

The use of nasal continuous positive airway pressure to treat upper airway obstruction in sleep has been the subject of patents, and has been referred to in a variety of medical publications and was developed primarily for adult use. In recent years nasal CPAP has been used in the treatment of infantile obstructive apnea. However, a major problem with effectively treating an infant subject with CPAP is obtaining a mask that is appropriate for that infant. There are two issue which are critical in the effective delivery of CPAP. First, the mask must be able to maintain a known pressure in the airways during both the inspiratory and the expiratory cycle. To do so requires a hermetic seal between the mask proper and the subjects skin. Secondly, it is necessary, or at least highly desirable, to minimise and eliminate torsional movement causing twisting of the mask and consequently the leaking that arises either from movement of the subject's head, or movement of the air delivery pipe which must be attached to the mask.

The extent to which these two issues affect the use of the mask in adults as opposed to infants varies considerably. First, in the case of achieving a good seal, the adult is able to readily adjust their mask if they are experiencing a less than perfect seal (which usually results in a leak of air from the mask). In contrast, infants are unable to manage the required readjustment themselves. Secondly, problems arising from the torsional effects between the two interfaces of the air delivery pipe to the mask manifold, and the mask seal to the face are greatly exaggerated with infants as compared with adults. It is well known that a typical infant makes many more movements, particularly head movements, during sleep compared with a typical adult. In particular, the infant has many twitches and startles with concomitant head movements during dreaming sleep (rapid-eye movement sleep, known as REM). Further, the infant spends much more time in REM sleep (up to 50% of total sleep time) than the adult (less than 25% or total sleep time). Clearly the greater the number of movements, the more likely it is that the torsional effect will cause the mask to lift from the face which will lead to air leaking. Again, the adult is better able to adjust their mask to overcome this problem, than the infant.

Notwithstanding these issues, until now, infant masks have been developed on the basis of scaling down the adult mask to approximate to the infant face and nose. The problems with this scaling down process are threefold.

First, the adult nose and middle third of the face is very different in shape from that of the infant. The adult nose is more elongated than, and protrudes far more from the surface of the face compared to the infant nose which is relatively flat, with no bridge, with the nares (nostril passages) pointing outwards. Therefore in order to fit the adult nose the base of the mask has a triangular shape elongated in the vertical axis In contrast, with an infant, the width at the base of the nose approximates the height from the base of the nose (nares) to the apex of the nose (nasion). The "proportional shape" of the nasal area of an adult is rectangular compared with a square "proportional shape" for an infant In addition to this basic difference in proportional shape, the adult face has quite marked contours especially around the nose and cheek area which are absent in the infant. The adult mask must therefore have acute angles which accommodate these facial contours. Thus, when an adult mask is scaled down for an infant, not only are the proportions wrong for the infant nose and face, but the angles which are unnecessarily incorporated, inadvertently introduce a new problem. Because the infant has a relatively flat nose, and virtually no bridge, the angles promote formation of channels in the sealing margin of the mask, especially in the region of the nasal bridge.

Secondly, in adult mask designs, the straps of the head harness connect with lugs on the rigid manifold of the mask in the order of 20 mm away from the surface of the face to allow the mask to accommodate the height of the adult nose. Because of this, a potential fulcrum effect is created. In the adult this fulcrum effect is not as problematic as in the infant, not only because the adult is less mobile during sleep as discussed above, but also because the contours of the adult face and cheeks can offset this rise. In the infant, when the mask used is merely a scaled down adult mask, the elevation of the strap lugs above the face is about 12 mm. This by itself creates a potential fulcrum as it does in the adult, but the effect is enhanced by the fact that there is no offset from the infant cheek due to the smaller facial area. Consequently, the straps holding the mask in place come into contact with the side of the face in the infant, compared to the cheek in the adult.

Thirdly, because the attachment of the paediatnc mask to the face and head mimics that of the adult mask, the torsional forces are increased. The greater torsional effect is due to the decreased surface area of the mask face contact relative to the air delivery pipe. Thus relatively minor movements can result in sufficient torsional forces to cause movement at the interface between the mask and the infants face.

There are numerous published patents and patent applications which relate to masks for use in CPAP and for other gas supply applications They include US 5243971 (Sullivan et al). AU 42476/99 (ResMed Ltd). WO 98/18514 (Sleepnet Corp). US 5657752 (Landis et al) and US 5 560 354 (Berthon-Jones et al) which describes a combined mouth and nasal mask according to the preamble of claim 1 and is considered as closest prior art for the present application. However all of those specifications are directed to masks for adults and it is significant to note that despite nasal CPAP having been in use on infants for over twenty years no satisfactory infant mask has yet been proposed.

US 5419317 discloses a facemask including a cup-shaped body for enclosing the nose of the patient. There is no reference or disclosure that the mask is suitable for an infant. The drawings show a single embodiment of a mask and although Figure 16 appears to disclose a face mask having an aperture which suggests an aperture which is more trapezoidal than triangular, in fact, the Figure is distorted and it is clear that it is intended to show the same triangular aperture that is disclosed in the other Figures.

It is an object of the present invention to alleviate some or all of the above mentioned problems with the prior art and provide an improved mask which is particularly suited for infant facial structures. For the purposes of this specification the term infant or infant human includes premature/neonatal babies, newborn babies, infants and small children having infant facial profiles who may be older than one year, possibly aged up to eighteen months to about two years old. The shape of the infant's face is the significant factor, not the age of the child.

### Summary of the Invention

Thus in a first broad aspect of the present invention, there is provided a mask for supplying gas under pressure to the nasal airway of an infant human, including:
a manifold for supplying air to an aperture in the mask:
a support structure for supporting the manifold; and
a shaped membrane structure formed from a thin walled membrane extending generally away from the support structure, the shaped membrane structure defining an enclosure for receiving at least the nares of an infant human nose through an aperture wherein;
part of the membrane around the aperture is sufficiently flexible to mould to the shape of the infant human's nasal area or is contoured to generally match the contours around that nasal area whilst the membrane structure itself has sufficient rigidity to support the weight of the support structure without collapsing,
characterised in that the aperture is generally trapezoidal having a top, two at least partially straight sides and a base adapted to fit around the nasal area of the infant human wherein the ratio of the length of the top of the generally trapezoidal aperture to the length of the base of the generally trapezoidal aperture is between 1 to 1.8 to 1 to 3.

The provision of a generally trapezoidal rather than the generally triangular apertures for fitting around the nares provides a substantially improved fit when the mask is used with infants. The moulding or contouring of the membrane structure around the aperture to match the shape of the infant's facial contours around the nasal area is also important in ensuring a comfortable fit and an effective seal. edge of the generally trapezoidal aperture will typically be gently curved as will the base edge of the aperture although typically relatively less curved than the top. The isosceles trapezium also has a base sides and a top and preferably has angles between its base and sides of 55 to 65 degrees most preferably about 60 degrees and the ratio of the length of the top of the trapezium to the length of the base is between about 1 to 1.8 to about 1 to 3 and most preferably 1 to 2.4.

In a particularly preferred embodiment, the backing plate is generally triangular in shape.

Typically, the thickness of the membrane will diminish from the backing plate to the aperture. The membrane may be about 1.2 mm thick adjacent the backing plate but only 0.2 mm thick at the aperture.

It is preferred that the backing plate is flexible.

Typically, a flexible arm extends away from the backing plate at or adjacent each of the three apexes of the triangular plate.

It is preferred that a pad is defined at the end of each flexible arm distal from the plate.

In a particularly preferred embodiment, the pads, arms membrane and backing plate are all integrally moulded from a flexible elastomeric material, most preferably a high tear resistant silicone elastomer such as Silastic (Registered Trade Mark of the Dow Corning Corporation) or Santoprene (Registered Trade Mark of the Monsanto Co) at a thickness of between 3 to 6 mm, typically 3.5 mm.

The moulding of the back plate and arms from a flexible elastomeric material, enables the arms to flex at the point where they meet the backing plate. This obviates the problems of the prior art in which rigid arms extend away from the mask (to which are attached straps) which increases the torque applied to the mask. The mask of the present invention, also allows the straps to pass over the infant's cheeks, rather than down the side of their heads. That provides a more secure fit of the mask and makes the mask less likely to move.

For a mask for a typical three month old infant, the base of the triangle forming the backing plate will be approximately 40 mm long with the height of the triangle around 35 mm. The membrane will typically extend around 10 to 14 mm, typically 12 mm away from the backing plate. The generally trapezoidal aperture in the membrane will have a base of around 20 to 25 mm and a height of around 15 mm and a top which is approximately 12 mm long and which is preferably slightly curved

In a particularly preferred embodiment, the plane of the arms is offset from the plane of the backing plate by around 10 to 25°, such that when the mask is placed over the nasal area of an infant human the arms tend to extend downwardly onto the cheeks of the infant. Preferably as well as the offset from the plane of the backing plate, those arms which extend towards the cheeks of the infant also extend down the infants face so as provide a force vector (when the strap is attached to a head harness) which tends to pull the mask down from the nose bridge preventing the mask form moving up in that direction. Infants tend to move their head from side to side tends to cause existing masks to ride up the infant's face towards their forehead. The preferred embodiment described above addresses this problem.

The arms may be attached to an infant or human face by use of skin adhesive. Alternatively, the pads may be connectable to straps attached to a cap. These straps could be connected to the pads by fastening materials such as velcro or the like.

Typically, a number of small holes will be defined on the manifold to provide a constant leak to atmosphere and thus a way out for expired air. The number and size of the holes are determined by the pressure of air supplied to the mask and flow delivery system and are chosen to enable the desired pressure and air flow through the mask.

In one embodiment of the invention, the mask is provided in two detachable parts.

The separation of the mask system into two detachable parts, readily allows for the first part or applicator to be put in place around the nose of an infant and to be held in place with or by adhesive or by straps and a cap. The infant or child can then be allowed to sleep without the presence of air pressure and air flow coming into the nasal region Thus the applicator can be used as a trainer to get the child or infant used to the mask. Further, once the infant or child is asleep, the parent or carer can then snap connect the air delivery to the applicator completing the formation of a fully operational CPAP mask or pressure support ventilation mask

In a particularly preferred embodiment, engagement is by means of a generally tubular projection fitting inside a generally cylindrical hole, the hole being provided on the manifold and the tube on the backing plate, vice versa.

It is preferred that one of the aperture or the projection is generally elliptical so that compression of the ellipse into a circle can be used for inserting the projection into the hole such that when the ellipse is relaxed, it will return to its original shape and retain the projection in the aperture.

In an alternative embodiment, the manifold is integral with the backing plate, but the structure is such that the manifold extends along one of the arms of the mask, typically the arm which extends from the mask towards the patient's forehead, in use.

Preferably the manifold extends to one distal end of one of the arms where a port or hole for receiving a gas delivery pipe is provided.

The provision of the air port at the end of one of the arms of the mask greatly reduces the torsion effects on the mask due to the air delivery pipe.

The manifold may be made of a thin flexible material and include a series of hoops or strengthening rings to assist the membrane in maintaining the shape of the manifold. The arm and manifold are then sufficiently flexible to absorb some of the movement or the mask relative to the delivery pipe.

### Brief Description of the Drawings

The invention will now be described by way of example only and with reference to the accompanying drawings in which:
Figure 1 is a isometric view of a first embodiment of a mask to fit the nasal area of a three month old infant for supplying air at a positive pressure to the infant's nasal airways;
Figure 2 is a front view of the mask shown in Figure 1;
Figure 3 is a top view of the mask shown in Figure 1;
Figure 4 is a side view of the mask shown in Figure 1;
Figure 5 is a rear view of the mask shown in Figure 1;
Figure 6 is an bottom view of the mask shown in Figure 1;
Figure 7 shows a section cut through the centre of the mask of Figure 1. showing the lower part of the mask only:
Figure 8 shows the shape of an opening in the mask of Figure 1;
Figure 9 illustrates a first contour generated for shaping the infant mask of Figure 1:
Figure 10 illustrates a second contour generated for shaping the infant mask of Figure 1:
Figure 11 illustrates a contour geometry representing an infant's facial surface used in the design of the infant mask of Figure 1:
Figure 12 is a front view of a second embodiment of a mask to fit the nasal area of a three month old infant:
Figure 13 is an isometric view of a third embodiment of a mask to fit the nasal area of a three month old infant.
Figure 14 is a side view of the mask of Figure 13. and
Figure 15 is an isometric view of a variant of the mask of Figure 1 fitted to a harness.

### Detailed Description of Preferred Embodiments

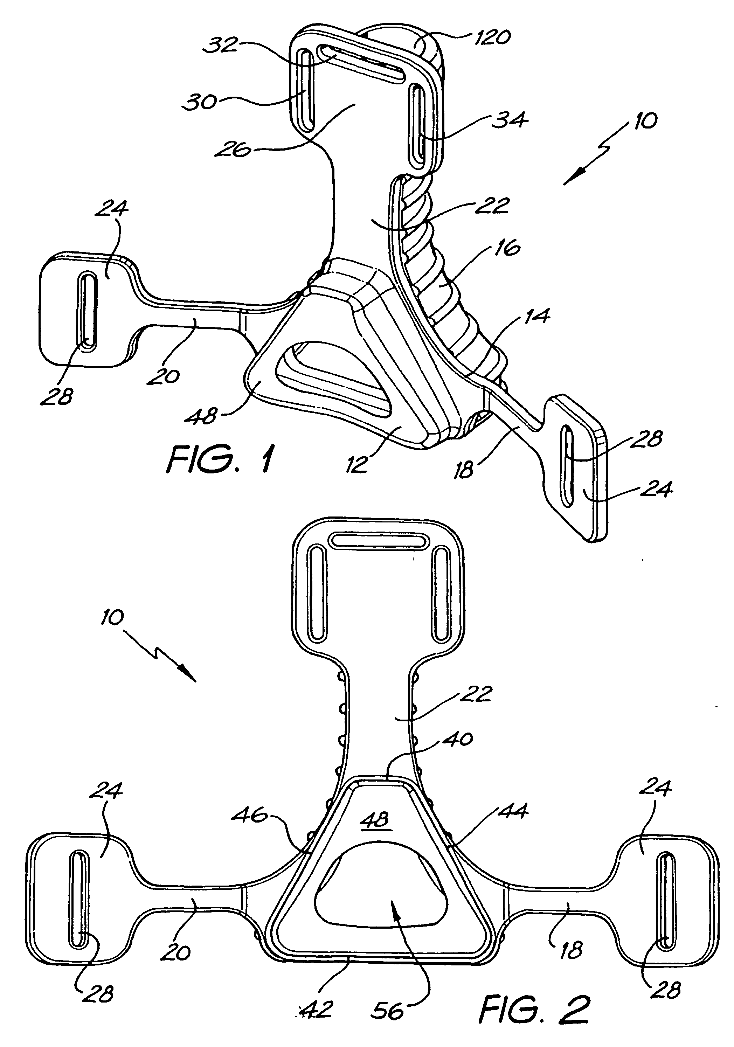

Referring to the drawings. Figure 1 shows a mask 10 suitable for use on three month old infants. In the described embodiment the mask is made from three moulded components a shaped membrane structure 12, a support structure 14 in the form of a combined support or backing plate and arms 14 and a manifold 16 which are glued together, although other constructions are possible. The components of the mask are preferably made from Silastic (Registered Trade Mark of the Dow Corning Corporation). However, other flexible or elastomeric materials could be used.

The centre of the support structure is a generally triangular plate from which three arms 18, 20 and 22 extend. The support structure is about 4mm thick and is flexible but will retain its shape and is not floppy.

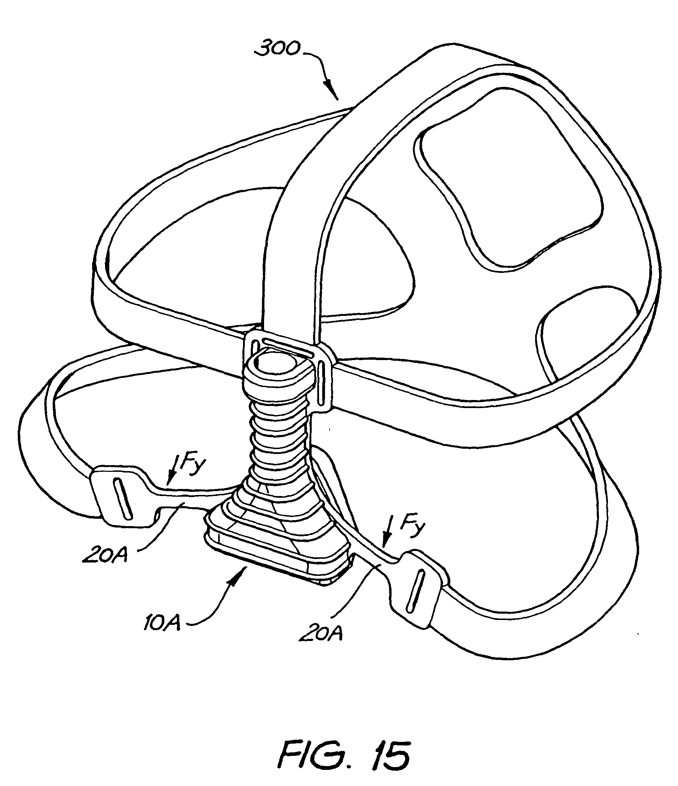

One of the arms 22 extends away from the top apex is about 40mm long and is wider than the other two arms 18 and 20 which extend away from the triangular plate adjacent each of the two apexes at the base of the triangular plate as oriented in Figures 1 and 2. At the end of each of the two arms, there is a rectangular pad 24 measuring approximately 15 x 17 mm. A relatively larger pad 26 is defined at the end of arm 22. Each pad 24 defines a slot 28. The larger pad 26 includes three slots 30, 32, 34. The slots are provided to allow a head harness to be attached to the mask for securing the mask in place on an infant's face. Figure 15 illustrates this with a variant of the mask shown in Figure 1. As discussed above, the pads, arms and triangular plate of the support structure are all preferably integrally moulded in one component.

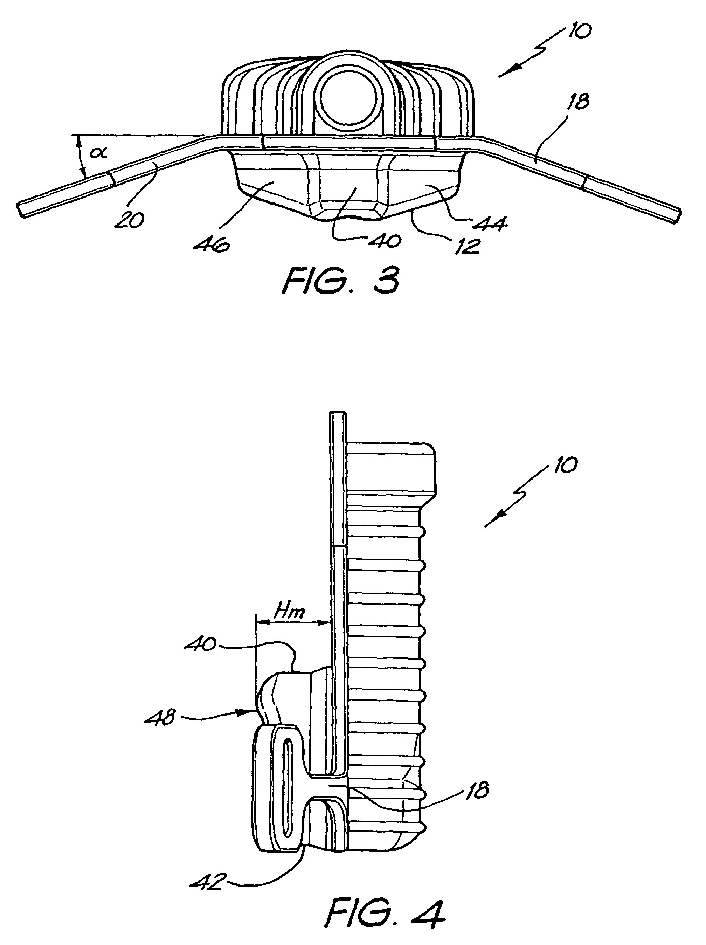

With reference to Figure 3, it can be seen that the plane of the arms is offset from the plane of the triangular backing plate by an angle alpha of around 20°. This offset is best seen in Figure 3. Thus when the mask is located on an infant's face the arms extend down towards the infant's cheeks so that the mask has a low profile and tends to fit better and is less likely to be dislodged during sleep.

The shaped membrane structure 12 is generally trapezoidal in cross section and is glued to (or may be integrally moulded with) the perimeter of the triangular backing plate. It comprises a thin silastic membrane and as oriented in Figure 2 has four walls, namely a top 40, a base 42, and sides 44 and 46. As can be seen in Figure 3 the walls extend away from the triangular backing plate, with the sides 44 and 46 tapering inwardly generally at an angle of approximately 70 to 80° relative to the plane of the back plate.

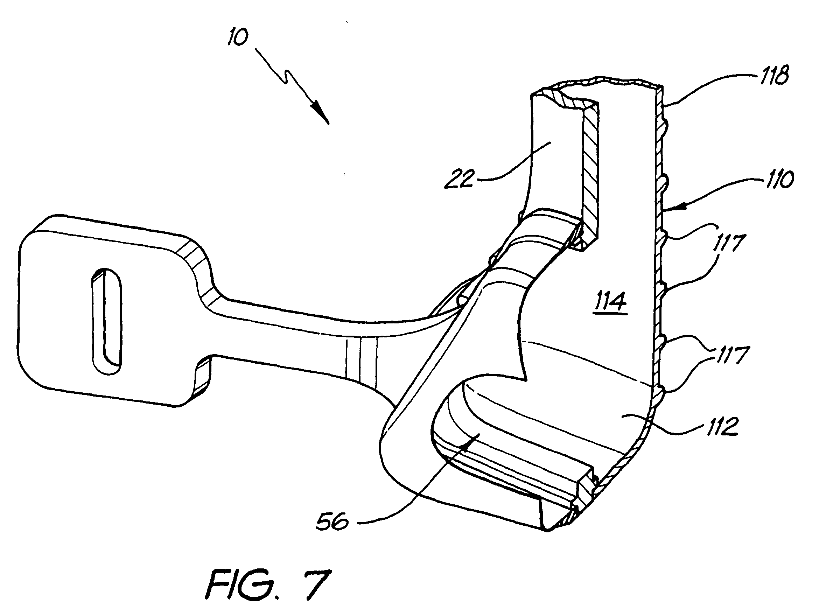

The height of the membrane Hm above the backing plate is approximately 12 mm (although this height may vary from 7mm upwards depending on the size of the infant human)and the height is not constant as the front face 48 of the membrane structure is contoured. The sides of the membrane vary in thickness from about 1.2mm where the structure joins the backing plate to about 0.2mm at the front face 48. This is best seen in the section. Figure 7

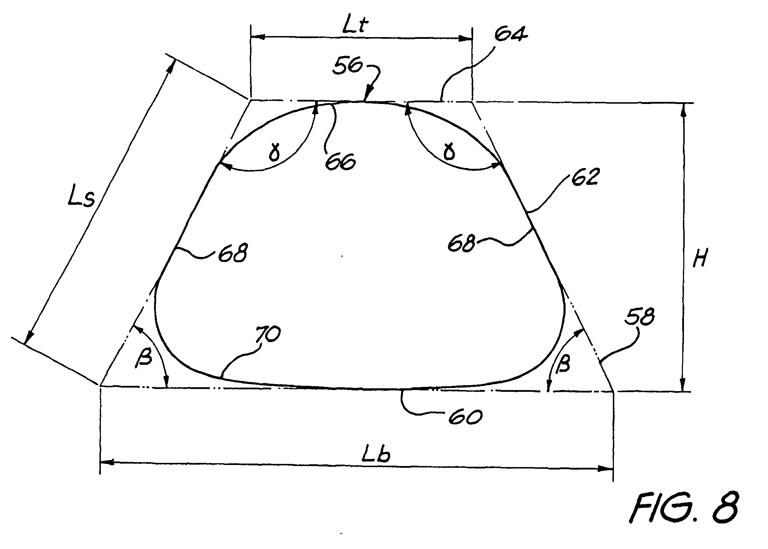

A generally trapezoidal aperture 56 is defined in the front face 48 of the membrane structure. The shape and size of the aperture is shown schematically in Figure 8 located inside an isosceles trapezium 58 shown in phantom which touches the sides, top and base of the aperture, although the membrane may be scaled up or down depending on the age/size of the infant for which the mask is made and the proportions may be varied whilst still retaining the benefits of the invention. The angle beta between the base 60 and the sides 62 of the trapezium is about 61 degrees. The length of the base Lb is about 29.5 mm. The height H of the trapezium is about 15.5mm. The length of the sides Ls is about 17.5mm and the length Lt of the top is about 12.5mm. The angle gamma between the top and the sides is 119 degrees. The ratio Lt to Lb is in the described embodiment 1 to 2.4. although it is envisaged that ratios of between about 1 to 1.8 to about 1 to 3 could be used. The rim or edges of the aperture define the contact area around the nasal area of an infant, in use, as can be seen the aperture itself has rounded sides with the upper edge 66 of the aperture which contacts the bridge of the nose of the infant is curved. The side edges 68are generally straight although the corners where they meet the upper edge 66 and the lower edge 70 are rounded. The lower edge 62 where, in use, the structure contacts the skin area at the below the nares and above the infant's top lip is longer than the upper edge and is also curved but more gently than the upper edge 64.

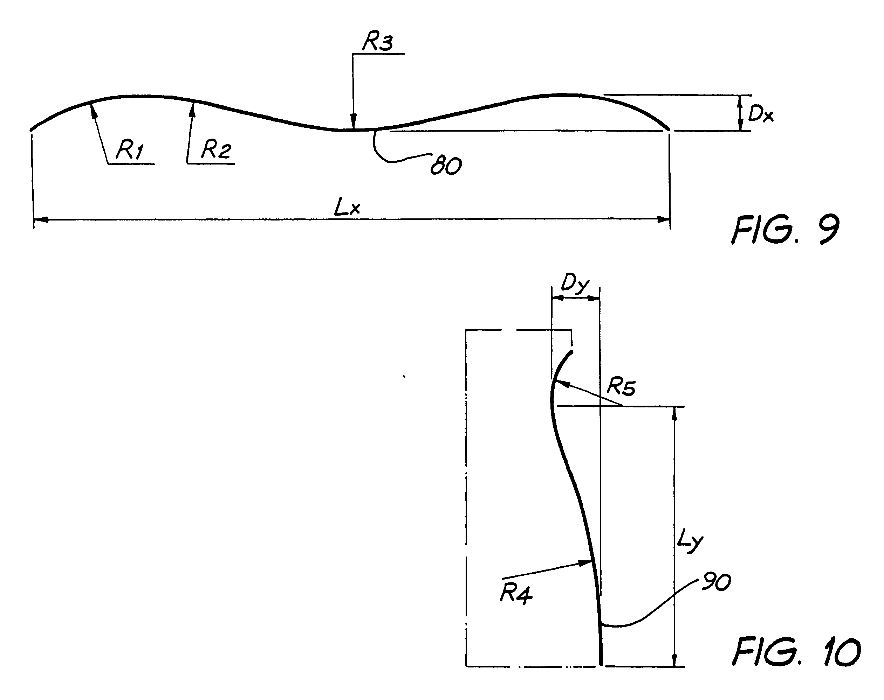

As well as having an aperture which is optimised to suit an infant the front face 48 of the membrane structure is also contoured to suit an infant. The described embodiment is for a three month infant. Figure 9 illustrates an "x-axis" contour line or spline curve 80 which is used in conjunction with a Y axis contour line/spline curve 90 to simulate the shape of a typical infant's facial contours The curve 80 is 42mm long Lx and the radii R1, R2 and R3 as shown are 11.6mm, 46.2mm and 17 70mm, respectively The depth of the curve between the apices is 2.2mm. The curve simulates the contour across an infant's face. For the y axis curve 90 shown in Figure 10 R4 is 62.4mm R5 is 80mm, the depth of the curve between apices is 5.4mm and the length Ly of the curve between the apices is 27.0mm. Clearly the shapes of the curves can be varied from the described embodiment whilst still retaining the benefits of the invention.

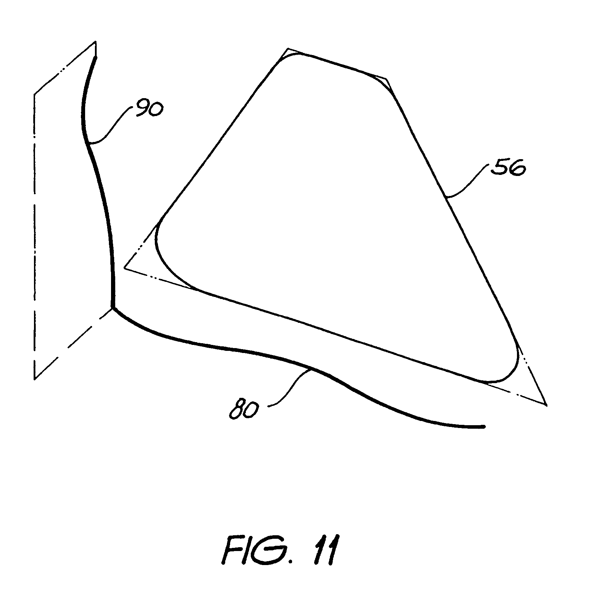

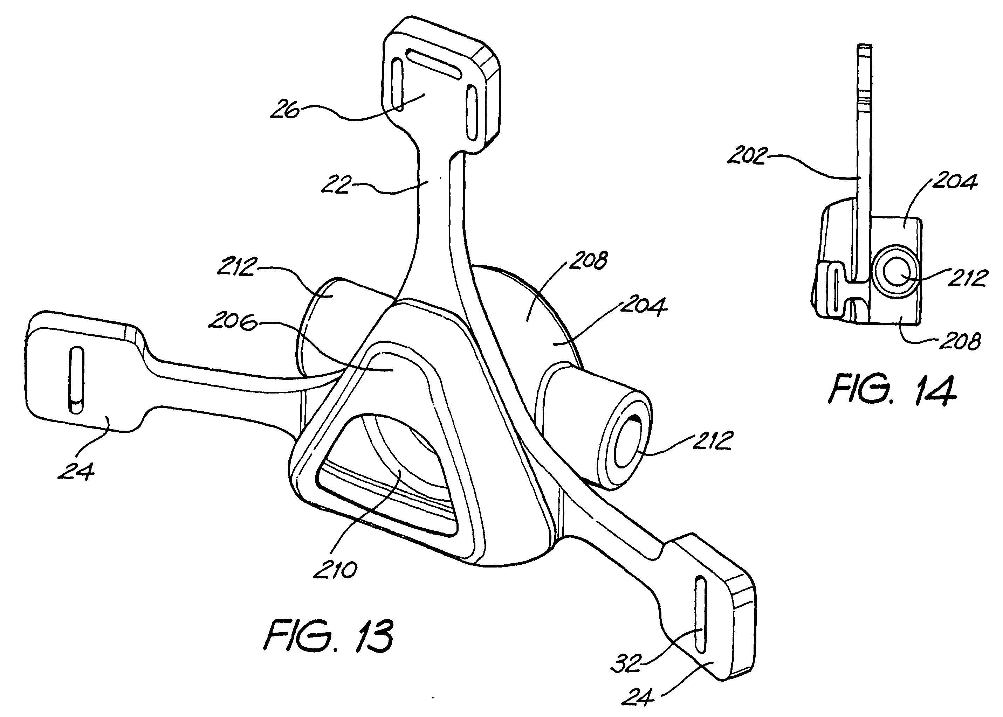

Figure 11 illustrates the use of the curves 80 and 90 to contour the front face 48 of the aperture profile 56 with contour 90 being swept along contour 80. This creates an appropriate bubble to infant contact surface that does not rely significantly on flexing or deformation of the membrane structure to achieve a good seal and a comfortable fit. It is possible to have a generally flat front 48 as shown in the variant illustrated in Figure 13 for example. The silastic material is such that when air is supplied at the aperture it forms a hermetic seal by distending the membrane and enhancing the form of the membrane to the facial contours of the infant. However with a flat front face one is much more reliant on the membrane structure deforming and moulding to the infant's facial contours hence the shaped membrane is much preferred and has substantial advantages over a planar front face.

The shape of the aperture provides for a good fit over the nasal area of an infant and allows for unimpeded breathing of air by that infant. Further the thickness of the sides of the membrane at the backing plate, the inwards tapering of the sides and the shape of the membrane structure generally, gives the membrane structure the necessary rigidity which is necessary for it to function and maintain its shape. It is important that when the device is placed over an infants nose, the membrane structure has sufficient strength and rigidity to prevent it collapsing under the weight of the backing plate and any air or oxygen tubes attached thereto However the membrane can still be as thin as 0.2 mm at the infant's face to enable it to mould to the shape of that infants face around its nose if necessary.

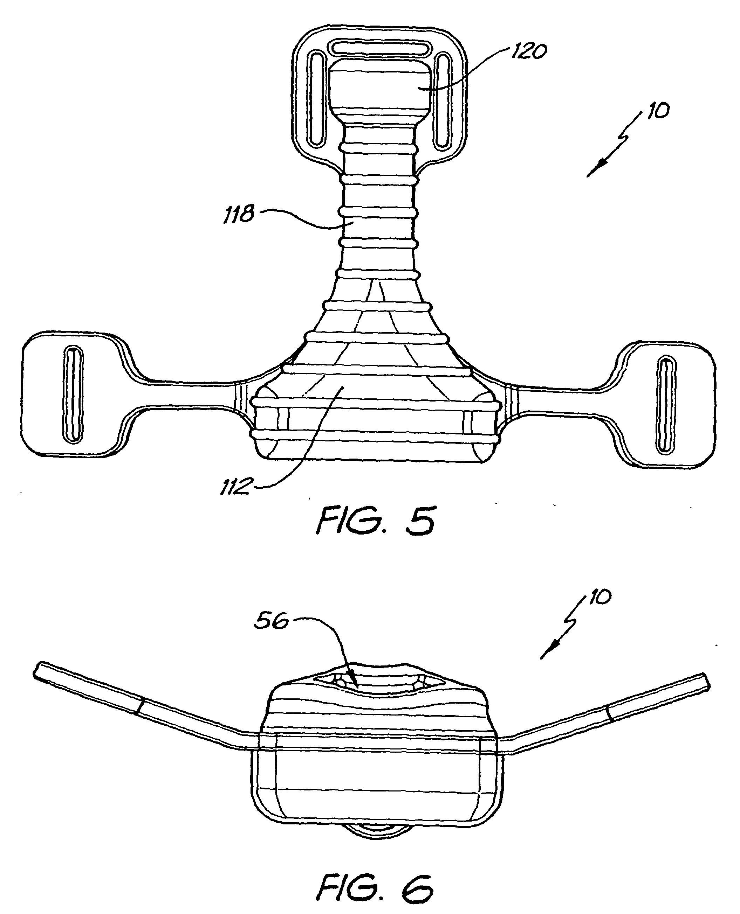

The manifold 16 is best seen in Figures 5 and 7. It includes a generally triangular portion 112 with a back 114 and an open face c which is in fluid communication with the membrane structure and aperture 56. A channel portion 118 extends away from the triangular portion. The manifold is formed from a thin membrane approximately 1 mm thick reinforced with a series of thicker ribs 117 The manifold is glued to the reverse side of the support structure 14 in a gastight fashion as is best seen in Figures 7. The arm 22 and channel 118 combine to form a pipe leading from the distal end of the arm 22 to the centre of the mask. An air inlet port 120 is defined at the end of that pipe. Pressurised air travels down the pipe through an aperture in the support structure into the membrane structure and out via the aperture 56. The manifold 110 also includes a number of small holes(not shown) which allow the leakage to atmosphere of expired air.

The structure formed from the membrane and strengthening ribs allows the arm to flex but at the same time, maintain the integrity of the manifold. The upper pad can be anchored to the forehead of the infant with the air delivery pipe attached. The torsion acting on the mask due to the pipe is concentrated at this point. This has substantial benefits, as it reduces the amount of torsion acting on the mask and helps leakage of the face mask in use.

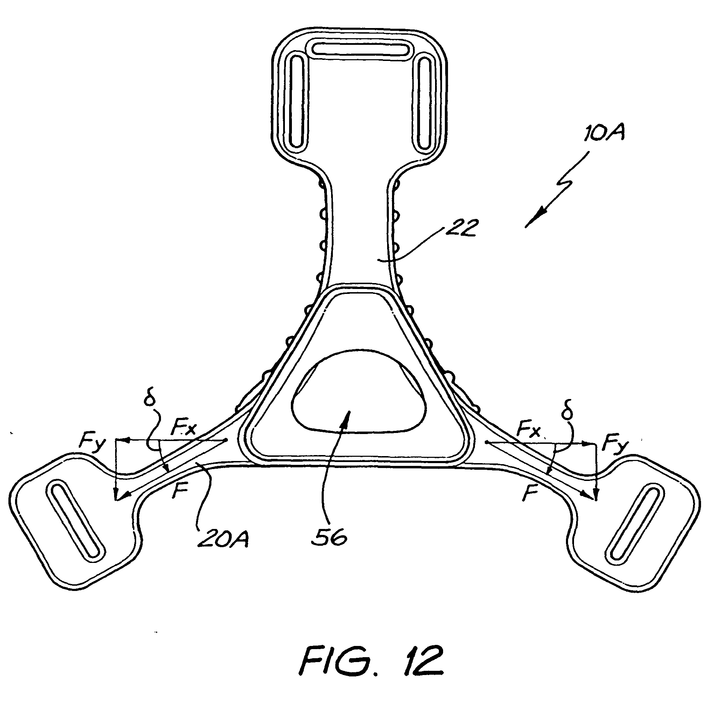

Figure 12 shows a variant 10A of the mask of Figure 1 in which the lower arms 18A and 20A do not extend generally parallel to the base of the triangular plate but are angled downwardly. This enables the pads to locate lower down on an infant's face which generally provides a better fit than the mask of Figure 1. Forces F acting along the arm 20A provide a force vector Fy downwards as well as a vector Fx across the infants face(when the strap is attached to a head harness). The vector Fy tends to pull the mask down from the nose bridge preventing the mask form moving up in that direction. This is an important advantage because infants tend to move their head from side to side tends to cause existing masks to ride up the infant's face towards their forehead. As illustrated the angle delta between the base of the mask and the angle of the arms is about 30 degrees and may preferably be about 20 degrees to about 40-45degrees.

Figures 13 and 14 show a further variant in which a mask 200 is separable into two parts, an "applicator" 202 and a manifold 204 best seen in Figure 14. The applicator is similar to the front part of the mask of Figure 1 except that the front face 206 of the membrane structure is flat. The rear of the mask is different since air is supplied via the detachable manifold 204

The manifold 14 includes a circular chamber 208 having at least one connector port 210, the specific embodiment having two opposed connector ports 212, one of which is normally closed with a bung, in use. The connector ports 212 allows the attachment of an air delivery pipe. The chamber 60 defines an open end from which an annular cylinder 66 projects and defines an external flange which is shaped and configured to engage behind an internal flange of the applicator for uses in connecting the manifold to the applicator. Other connection means could be used.

In use, the applicator may be used without the manifold in place on an infant to train the infant to use a mask and to acclimatise the infant to the feel of a mask on there face without the manifold and tubes which are bulky. The use of nasal CPAP or nasal ventilation or nasal pressure support systems in infants, often requires a period of training in which the infant or child is allowed to wear part of a mask at bed time, and during sleep, before any attempt is made to introduce the air flow and pressure source. The mask system of the present invention allows for this in a very satisfactory manner since the use of the applicator alone will not impede the flow of air to the infant. The applicator may be stuck to a child's face by applying skin adhesive such as elastogel or Duo Derm (manufactured by Convatec Bristol Myers Squibb) over the pads 32. Alternatively, the pads could define further attachment means for connecting to straps attached to a cap.

Further, the infant or child can be allowed to go asleep with only the applicator attached without the presence of air pressure and air flow coming into the nasal region, but when asleep, the carer or parent can then snap fit the manifold to the applicator so completing the formation of a fully operational CPAP mask, or pressure support ventilation mask. Typically in use, the system should provide fresh air at a mask pressure of approximately 5 mm water.

In a variation (not illustrated) either one of the aperture in the applicator or the projection from the manifold is generally elliptical so that compression of the ellipse into a circle can be used for inserting the projection into the hole such that when the ellipse is relaxed, it will return to its original shape and retain the projection in the aperture.

Figure 15 illustrates the mask 10A shown in Figure 12 attached to a harness 300. The Figure illustrates the force vector Fy along the arm 20A pulling the mask down the infant's face. Infants move their heads from side to side in sleep, since they are not strong enough to lift their heads and in existing masks which do not provide the downwards vector, this movement causes the mask to ride up the infant's forehead.

One advantage of the embodiments of the present invention, is that the applicator fits to an infant's face mostly and through its flexibility can effectively approximate to the infants facial structures. By fitting more closely and in particular, since the depth of the shaped membrane is quite shallow, the height of the mask is above the infants face is minimised and this reduces the fulcrum effect which is a particular problem with the prior art systems described above. Reducing the fulcrum effect is particularly important with infants, as they firstly tend to move around in their sleep a lot more than adults, and secondly are incapable of readjusting their mask if the mask leaks.

Embodiments of the present invention allow easy connection and disconnection from an air delivery system.

Although of the embodiments of the present invention have been described as comprising a mask made of silastic, it should appreciated that it would be possible to use other elastomeric materials which have similar properties to silastic.

Whilst the masks described in the specific description are configured for a 3 month old infant, the design is such that by selecting the size of the square upon which the mask is based, a wide range of mask sizes can be created that will readily accommodate the continual growth and change in facial measurement during the first two years of life. Further it would also be possible to use various of the features of the infant masks described above in masks for older children and even adults.

## Claims

1. A mask (10) for supplying gas under pressure to the nasal airway of an infant human, including:
a manifold (16) for supplying air to an aperture (56) in the mask:
a support structure (14) for supporting the manifold (16); and
a shaped membrane structure (12) formed from a thin walled membrane extending generally away from the support structure (14), the shaped membrane structure defining an enclosure for receiving at least the nares of an infant human nose through an aperture wherein;
part of the membrane around the aperture (56) is sufficiently flexible to mould to the shape of the infant human's nasal area or is contoured to generally match the contours around that nasal area whilst the membrane structure itself has sufficient rigidity to support the weight of the support structure without collapsing,
**characterised in that** the aperture (56) is generally trapezoidal having a top (64), two at least partially straight sides (62) and a base (60) adapted to fit around the nasal area of the infant human wherein the ratio of the length of the top of the generally trapezoidal aperture to the length of the base of the generally trapezoidal aperture is between 1 to 1.8 to 1 to 3..

2. A mask as claimed in Claim 1 wherein the shaped membrane structure has a generally flat front face formed from a portion of the thin walled membrane, the generally flat face defining a contact area (48) which in use contacts that part of an infant's face around that infant's nares and wherein the contact area is profiled or contoured to approximate to the profile of that part of the infant's face which it is to contact in use and wherein the generally trapezoidal aperture is defined in the flat front face.

3. A mask as claimed in claim 1 or claim 2 wherein the ratio of the length of the top (64) of the generally trapezoidal aperture (56) to the length of the base (60) of the generally trapezoidal aperture is 1 to 2.4.

4. A mask as claimed in any preceding claim wherein the thickness of the membrane diminishes from the support structure to the aperture.

5. A mask as claimed in any preceding claim wherein the support structure (14) is flexible and generally planar, and wherein an integrally formed flexible arm (18;20;22) extends away from the support structure at or adjacent each of the three apexes of the triangular plate.

6. A mask as claimed in claim 5 wherein a pad (24;26) is defined at the end of each flexible arm distal from the support structure, and wherein the pads (24), arms (18;20;22), membrane (12) and support structure (14) and manifold (16) are all moulded from a flexible elastomeric material,

7. A mask as claimed in any one of claims 5 to 6 wherein the plane of two of the arms (18;20) adjacent the base of the mask is offset from the plane of the support structure by around 10 to 25°, such that when the mask is placed over the nasal area of an infant human the arms tend to extend downwardly onto the cheeks of the infant.

8. A mask as claimed in any one of claims 5 to 7 wherein the manifold extends to a distal end of one of the arms (22) where a port (120) for receiving a gas delivery pipe is provided and wherein the manifold comprises a thin flexible material supported by a series of spaced relatively thicker support ribs.

9. A mask as claimed in any one of claims 7 to 8 wherein two of the arms adjacent the base of the mask make an angle of 20 to 45 degrees, with the base of the mask.

10. A mask as claimed in any one of claims 1 to 9 wherein the generally trapezoidal aperture (56) defines corners which are rounded.

11. A mask as claimed in any one of claims 1 to 10 wherein the top (64) of the generally trapezoidal aperture (56) is curved.

## Patentansprüche

1. Maske (10) zur Druckgaszufuhr zum Nasenweg eines Säuglings, mit Folgendem:
einem Verteiler (16) zur Zufuhr von Luft zu einer Öffnung (56) in der Maske;
einer Stützkonstruktion (14) zur Abstützung des Verteilers (16); und
einer geformten Membrankonstruktion (12), die aus einer dünnwandigen Membran gebildet ist, die sich von der Stützkonstruktion (14) allgemein weg erstreckt, wobei die geformte Membrankonstruktion eine Umhüllung zur Aufnahme mindestens der Nasenlöcher der Nase eines Säuglings durch eine Öffnung definiert; wobei
ein Teil der Membran um die Öffnung (56) herum flexibel genug ist, um die Form des Nasenbereichs des Säuglings zu formen, oder so konturiert ist, dass er den Konturen um den Nasenbereich herum allgemein entspricht, während die Membrankonstruktion selbst eine ausreichende Steifigkeit besitzt, um das Gewicht der Stützkonstruktion aufzunehmen, ohne zusammenzusacken,
**dadurch gekennzeichnet, dass** die Öffnung (56) allgemein trapezförmig ist und eine Oberseite (64), zwei zumindest teilweise gerade Seiten (62) und eine Basis (60), die so ausgeführt ist, dass sie um den Nasenbereich des Säuglings herum passt, aufweist, wobei das Verhältnis der Länge der Oberseite der allgemein trapezförmigen Öffnung zur Länge der Basis der allgemein trapezförmigen Öffnung zwischen 1 bis 1,8 und 1 bis 3 liegt.

2. Maske nach Anspruch 1, bei der die geformte Membrankonstruktion eine allgemein flache Vorderseite aufweist, die aus einem Teil der dünnwandigen Membran besteht, wobei die allgemein flache Seite einen Kontaktbereich (48) definiert, der im Gebrauch den Teil des Gesichts eines Säuglings um die Nasenlöcher herum berührt, und wobei der Kontaktbereich so profiliert oder konturiert ist, dass er sich dem Profil des Teils des Gesichts des Säuglings, den er im Gebrauch berühren soll, annähert, und wobei die allgemein trapezförmige Öffnung in der flachen Vorderseite definiert ist.

3. Maske nach Anspruch 1 oder 2, bei der das Verhältnis von der Länge der Oberseite (64) der allgemein trapezförmigen Öffnung (56) zur Länge der Basis (60) der allgemein trapezförmigen Öffnung 1 zu 2,4 beträgt.

4. Maske nach einem der vorhergehenden Ansprüche, bei der die Dicke der Membran von der Stützkonstruktion zur Öffnung hin abnimmt.

5. Maske nach einem der vorhergehenden Ansprüche, bei der die Stützkonstruktion (14) flexibel und allgemein planar ist und bei der sich ein integral geformter flexibler Arm (18; 20; 22) am oder neben jedem der drei Scheitel der dreieckigen Platte von der Stützkonstruktion weg erstreckt.

6. Maske nach Anspruch 5, bei der ein Polster (24; 26) am Ende jedes flexiblen Arms distal von der Stützkonstruktion definiert ist und bei der die Polster (24), die Arme (18; 20; 22), die Membran (12) und die Stützkonstruktion (14) und der Verteiler (16) alle aus einem flexiblen elastomeren Material geformt sind.

7. Maske nach einem der Ansprüche 5 bis 6, bei der die Ebene zweier der Arme (18; 20) neben der Basis der Maske um ca. 10 bis 25° von der Ebene der Stützkonstruktion versetzt ist, so dass die Arme dazu neigen, sich nach unten auf die Wangen des Säuglings zu erstrecken, wenn die Maske über den Nasenbereich eines Säuglings angeordnet ist.

8. Maske nach einem der Ansprüche 5 bis 7, bei der sich der Verteiler zu einem distalen Ende eines der Arme (22) erstreckt, wo ein Kanal (120) zur Aufnahme eines Gaszufuhrrohrs vorgesehen ist, und bei der der Verteiler ein dünnes flexibles Material umfasst, das durch eine Reihe von beabstandeten, im Verhältnis dickeren Stützrippen gestützt wird.

9. Maske nach einem der Ansprüche 7 bis 8, bei der zwei der Arme neben der Basis der Maske mit der Basis der Maske einen Winkel von 20 bis 45 Grad bilden.

10. Maske nach einem der Ansprüche 1 bis 9, bei der die allgemein trapezförmige Öffnung (56) abgerundete Ecken definiert.

11. Maske nach einem der Ansprüche 1 bis 10, bei der die Oberseite (64) der allgemein trapezförmigen Öffnung (56) gekrümmt ist.

## Revendications

1. Masque (10) pour envoyer du gaz sous pression dans le conduit nasal d'un enfant, comprenant :
un collecteur (16) pour amener de l'air à une ouverture (56) pratiquée dans le masque;
une structure de support (14) pour supporter le collecteur (16); et
une structure de membrane configurée (12) formée à partir d'une membrane à paroi mince s'étendant essentiellement en s'éloignant de la structure de support (14), la structure de membrane configurée définissant une enceinte destinée à recevoir au moins les narines du nez d'un enfant à travers une ouverture prévue dans celle-ci;
une partie de la membrane autour de l'ouverture (56) est suffisamment flexible pour être moulée à la forme de la région nasale de l'enfant ou qui est profilée pour s'adapter d'une manière générale aux contours autour de la région nasale, alors que la structure de membrane elle-même présente une rigidité suffisante pour supporter le poids de la structure de support sans être écrasée,
**caractérisé en ce que** l'ouverture (56) est essentiellement trapézoïdale et présente un sommet (64), deux côtés au moins partiellement droits (62) et une base (60) adaptés pour s'agencer autour de la région nasale de l'enfant, le rapport de la longueur du sommet de l'ouverture essentiellement trapézoïdale et de la longueur de la base de l'ouverture essentiellement trapézoïdale étant compris entre 1:1,8 et 1:3.

2. Masque selon la revendication 1, dans lequel la structure de membrane configurée présente une face avant essentiellement plate formée à partir d'une partie de la membre à paroi mince, la face avant essentiellement plate définissant une zone de contact (48) qui, lors de l'utilisation, entre en contact avec la partie du visage de l'enfant qui se trouve autour des narines de celui-ci, et dans lequel la zone de contact est profilée ou conformée de manière à se rapprocher du profil de cette partie du visage de l'enfant avec laquelle elle est en contact lors du port du masque, et dans lequel l'ouverture essentiellement trapézoïdale est définie dans la face avant plate.

3. Masque selon la revendication 1 ou la revendication 2, dans lequel le rapport de la longueur du sommet (64) de l'ouverture essentiellement trapézoïdale (56) et de la longueur de la base (60) de l'ouverture essentiellement trapézoïdale est compris entre 1 et 2,4.

4. Masque selon l'une quelconque des revendications précédentes, dans lequel l'épaisseur de la membrane diminue à partir de la structure de support jusqu'à l'ouverture.

5. Masque selon l'une quelconque des revendications précédentes, dans lequel la structure de support (14) est flexible et essentiellement plane, et dans lequel un bras flexible formé intégralement (18; 20; 22) s'étend en s'éloignant de la structure de support à ou à proximité des trois sommets de la plaque triangulaire.

6. Masque selon la revendication 5, dans lequel une patte (24; 26) est définie à l'extrémité de chaque bras flexible à distance de la structure de support, et dans lequel les pattes (24), les bras (18; 20; 22), la membrane (12), la structure de support (14) et le collecteur (16) sont tous moulés à partir d'une matière élastomère flexible.

7. Masque selon l'une quelconque des revendications 5 à 6, dans lequel le plan des deux bras (18; 20) à proximité de la base du masque est décalé par rapport au plan de la structure de support d'environ 10° à 25°, de telle sorte que lorsque le masque est placé sur la région nasale d'un enfant, les bras ont tendance à se déployer vers le bas sur les joues de l'enfant.

8. Masque selon l'une quelconque des revendications 5 à 7, dans lequel le collecteur s'étend jusqu'à une extrémité distale d'un des bras (22) où un orifice (120) destiné à recevoir un tuyau d'alimentation de gaz est prévu, et dans lequel le collecteur comprend une matière flexible mince supportée par une série de nervures de support espacées relativement plus épaisses.

9. Masque selon l'une quelconque des revendications 7 à 8, dans lequel deux des bras situés à proximité de la base du masque forment un angle compris entre 20 et 45 degrés avec la base du masque.

10. Masque selon l'une quelconque des revendications 1 à 9, dans lequel l'ouverture essentiellement trapézoïdale (56) définit des coins qui sont arrondis.

11. Masque selon l'une quelconque des revendications 1 à 10, dans lequel le sommet (64) de l'ouverture essentiellement trapézoïdale (56) est courbe.
